# DEMANDE DE BREVET EUROPEEN

(11) **EP 0 855 185 A2**
(43) Date de publication de la demande: **29.07.1998**
(21) Numéro de dépôt: 98103971.2
(22) Date de dépôt: 22.02.1995
(51) Int. Cl.: A61K 48/00, C12N 15/86

(54) **Système hote-vecteur utilisable en thérapie génique**

(30) Priorité: 22.02.1994 FR 9401994; 15.12.1994 FR 9415135
(62) Demande divisionnaire de: 95910583.4
(71) Demandeur: UNIVERSITE PIERRE ET MARIE CURIE PARIS VI, 75252 Paris Cédex 05 (FR)
(72) Inventeur: Klatzmann, David, 75013 Paris (FR); Salzmann, Jean-Loup, 75005 Paris (FR)
(74) Mandataire: Becker, Philippe

(57) **Abrégé**

La présente invention concerne l'utilisation, pour la préparation d'une composition pharmaceutique destinée au transfert d'un transgène dans une cellule eucaryote, de séquences d'acides nucléiques dont la co-expression en trans dans ladite cellule eucaryote permet à ladite cellule eucaryote de produire des particules virales infectieuses et porteuses dudit transgène.

## Description

La présente invention est relative à une nouvelle approche pour la thérapie génique appliquée notamment à l'élimination de certaines catégories de cellules, telles les cellules tumorales ou certaines cellules infectées par des virus, par la production de rétrovirus recombinants à partir de nouvelles constructions génétiques.

Le concept de thérapie génique par gènes tueurs ou gènes suicides a été développé depuis 1986 par de nombreuses approches. Il implique l'expression d'un gêne, cette expression permettant la transformation d'une substance non toxique ou substance toxique par la cellule. Ce concept de thérapie génique est applicable et transposable à tout gène dont l'expression dans une cellule cible permet de transformer une substance inactive en substance active, ou vice versa, conduisant soit à une destruction (exemple du gène suicide), soit à une restauration de certaines fonctions.

Dans tout ce qui suit, l'application de ces concepts aux gènes suicides est plus particulièrement développée compte tenu de l'accessibilité de sa mise en oeuvre expérimentale. La transposition des moyens de l'invention à tout type de transgène dont l'expression conduit à modifier l'effet d'une substance dans un sens d'activation ou d'inactivation, est néanmoins à la portée de l'homme du métier.

La thymidine kinase du virus Herpès Simplex de type I (HSV1-TK) est l'enzyme qui a été l'objet du plus grand nombre d'investigations relatives aux gènes suicides.

Cette enzyme, atoxique pour les cellules eucaryotes, présente la caractéristique de pouvoir transformer certains analogues nucléosidiques tels l'aciclovir (ACV) ou la ganciclovir (GCV) en molécules monophosphatées, ce dont sont normalement incapables les kinases cellulaires (G.B. Elion, J. Antimicrob. Chemother. 12:9-17 (1983)). Ces nucléotides monophosphates sont ensuite convertis par des enzymes cellulaires en nucléotides triphosphates qui peuvent être utilisés lors de la synthèse de l'ADN et bloquer le processus d'élongation entraînant ainsi la mort de la cellule. L'analogue de nucléoside triphosphate n'est donc toxique que pour les cellules en division.

On conçoit donc tous les avantages qui peuvent être tirés de l'utilisation de ce type de toxines conditionnelles appliquée à la thérapie génique, notamment anticancéreuse.

En premier lieu, seule une toxicité conditionnelle permet de générer des clones cellulaires stables produisant les pseudo-particules virales capables de produire un tel transgène et transférer le gène suicide dans les cellules cibles. Il suffit en effet de cultiver ces cellules en l'absence d'ACV ou de GCV puisque la thymidine kinase de l'HSV1 n'est pas toxique pour la cellule en absence de ces drogues.

Ensuite, en cas d'effet secondaire du traitement, l'arrêt de l'administration de l'ACV ou du GCV fait cesser immédiatement la toxicité due au transgêne; en outre, un ajustement des doses de l'analogue nucléosidique permet de détruire sélectivement les cellules qui expriment fortement le transgène tout en préservant les cellules dans lesquelles le gène est faiblement exprimé ; cette toxicité restreinte aux cellules en division est un avantage important notamment pour le traitement des cellules cancéreuses.

Enfin, des données expérimentales in vitro et in vivo ont montré que des cellules n'exprimant pas HSV1-TK, mais au contact de celles-ci étaient également détruites par le traitement par l'ACV ("Coopération métabolique" ou "effet bystander")(Moolten F.L., 1986, cancer Research, 46:5276-5281 et Culver K.W. et al., 1992, Science 256:1550-1552).

Le mécanisme de cet effet reste encore incompris, mais il est possible que les analogues nucléosidiques triphosphatés puissent passer d'une cellule à l'autre par l'intermédiaire des "gap junctions".

Les rétrovirus apparaissent comme étant des vecteurs de choix pour transférer des gènes exogènes dans des cellules eucaryotes, notamment des cellules humaines.

Cependant, un préalable indispensable pour l'utilisation des rétrovirus à des fins thérapeutiques et notamment en thérapie génique est de s'assurer de la sécurité de leur utilisation.

Le danger principal de l'utilisation de rétrovirus en thérapie génique est la possibilité d'une dissémination d'un rétrovirus sauvage reconstitué dans la population cellulaire ou le tissu considéré.

Une telle prolifération pourrait conduire à des intégrations multiples du génome du rétrovirus dans le génome des cellules infectées pouvant conduire à tout type de dérèglement génétique.

Ce type d'approche pose donc le problème d'atteindre le plus possible de cellules cibles, et donc la capacité de ce système d'atteindre le plus possible de cellules tumorales, tout en assurant le contrôle de la prolifération de virus.

De nombreuses approches ont été développées jusqu'à présent pour mettre au point des cellules d'empaquetage ("packaging cell lines") qui produisent uniquement des rétrovirus défectifs, et porteurs du transgène d'intérêt ; le développement de telles lignées cellulaires a augmenté de façon considérable l'utilisation des rétrovirus en thérapie génique, grâce à la sécurité conférée par ces systèmes.

Les premiers vecteurs utilisés ont été des vecteurs rétroviraux portés par des particules pseudo-rétrovirales ayant une enveloppe amphotrope pour permettre leur utilisation dans différentes espèces, et défectifs pour contrôler leur dissémination. Le virus naturel de départ est généralement le virus murin de Moloney dont on a séparé les éléments agissant en cis et les éléments agissant en trans pour former deux génomes défectifs.

Le vecteur viral proprement dit a conservé les séquences cis indispensables : les LTR pour le contrôle de la transcription et de l'intégration, la séquence psi nécessaire pour l'encapsidation, la séquence PB nécessaire pour la réplication virale. Les gènes viraux (gag, pol, env) sont délétés et remplacés par le transgène à exprimer dans une cellule cible placée en principe sous son propre promoteur ou sous un promoteur jugé plus puissant ou régulable.

Les gènes rétroviraux gag, pol, env sont souvent intégrés dans un autre vecteur, parfois appelé Helper, défectif pour les séquences LTR et Psi (ψ). Leur expression permet l'encapsidation du transgène, à l'exclusion des gènes nécessaires à la multiplication du génome viral et à la formation de particules virales complètes.

La forme provirale du Helper est en général intégrée dans le génome d'une lignée cellulaire murine (par exemple fibroblaste NIH/3T3) qui joue à la fois un rôle d'hôte pour le vecteur et de Helper pour les fonctions qui lui font défaut. Après transfection du vecteur, la souche cellulaire devient capable de produire des particules virales infectieuses défectives. Cependant, ces particules ne contiennent uniquement que le gène à transférer (transgène) mais ne contiennent pas l'information nécessaire à la reconstitution de particules virales complètes dans les cellules cibles. Ce système est donc conçu pour empêcher toute propagation ultérieure de virus après la première infection, c'est-à-dire si le virus infectieux porteur du transgène pénètre dans une cellule dépourvue de l'information de type Helper (gag, pol et env), sa production est arrêtée.

Donc, classiquement, la lignée d'empaquetage ("packaging cell line") est une lignée cellulaire susceptible d'apporter l'information Helper en trans par rapport au génome viral présent dans la particule, de telle façon que des particules virales infectieuses défectives puissent se reconstituer.

Différents systèmes d'empaquetage permettant la production de virus recombinants défectifs ont été décrits dans les documents suivants :
- la demande de brevet EP 0 243 204 décrit l'utilisation de rétrovirus comme moyen d'empaqueter toute substance pour traverser la membrane de cellules cibles eucaryotes ;
- la demande de brevet WO 89/07150 décrit une cellule de packaging de rétrovirus, les deux plasmides exprimant en trans les différents gènes viraux (env, gag, pol), mais aucun des deux ne comprenant la séquence Psi (ψ) de packaging : la production de virus dans ces lignées est comprise entre 10⁴ et 10⁶ CFU/ml ;
- les demandes de brevets WO 90/02806, WO 90/12087, EP 0 476 953, WO 93/04167 et WO 93/10218 sont des publications décrivant des transferts de gènes in vivo par utilisation de vecteurs rétroviraux, eux-mêmes produits par des cellules d'empaquetage.

Les nombreuses références de constructions et d'applications citées dans ces brevets sont incluses à titre de références citées dans la présente demande.

Des développements récents ont conduit à utiliser un transfert de gènes in vivo en transférant directement les cellules d'empaquetage productrices de vecteurs pour le traitement de tumeurs ; l'élimination par exemple de tumeurs cérébrales expérimentales microscopiques par injection stéréotaxique de cellules produisant des rétrovirus TK-HSV1, puis traitement par le GCV, a été rapportée par Culver et col. (Science 256:1550-1552, 1992).

De la même façon, certains des auteurs de la présente demande ont montré que, chez le rat, le transfert in vivo du gène HSV1-TK par injection directe de fibroblastes murins produisant des particules rétrovirales recombinantes, conduit à une réduction considérable de tumeurs hépatiques chez le rat (Caruso M. et al., Proc. Natl. Acad. Sci. USA, 90:7024-7028, 1993 et Yves Panis et col., C.R. Acad. SC. Paris,, Tome 315, Série III, p. 541-544) ; ces articles mettent en outre en évidence une diminution très importante du nombre de cellules cancéreuses au niveau des tumeurs, sachant que, de façon surprenante, le nombre de cellules transduites in vivo par ce type de technique est sans doute inférieur à 10% pour TK-HSV1.

Cet effet pourrait s'expliquer par le mécanisme de coopération métabolique déjà évoqué plus haut.

Ce système de lignées d'empaquetage permettant de produire des particules virales porteuses d'un transgène est donc extrêmement prometteur pour utilisation en thérapie génique et notamment pour l'expression de gènes conditionnels.

Ces systèmes mis au point et décrits dans les nombreuses références citées plus haut présentent cependant certaines limites qui sont :
1) la faible productivité de ces lignées cellulaires (titre infectieux inférieur ou égal à 106 PFU/ml), le rétrovirus normal Mo MuLV produisant environ 108 PFU/ml et un virus comme l'adénovirus environ 109;
2) une deuxième limite de ce type de technique est qu'il met en oeuvre des lignées fibroblastiques NIH/3T3 qui sont des lignées adhérentes : ceci est un avantage lorsque l'on utilise le surnageant de cellules contenant les particules virales, mais par contre est un inconvénient lorsque l'on veut utiliser les lignées transfectées elles-mêmes à titre de médicament, les lignées injectées in vivo produisant alors les particules virales recombinées in situ :
3) un troisième inconvénient, qui est la contrepartie de l'avantage de sécurité du système, est que si les particules virales exprimées in situ par la lignée de packaging peuvent transférer le transgène d'intérêt dans les cellules en division, toute dissémination ultérieure est bloquée à ce niveau. Ceci limite l'efficacité du transfert du transgène du fait du blocage de la dissémination.

Il était donc important de concevoir un système permettant éventuellement d'auto-entretenir la production de particules virales recombinantes porteuses du gène d'intérêt tout en étant assurés de contrôler toute propagation ultérieure au moment choisi afin de préserver les conditions de sécurité requises, à savoir la maîtrise totale de la dissémination du virus sous sa forme sauvage.

Dans tout ce qui suit, le terme de "transgène" désigne le gène à exprimer dans la cellule cible, gène qui, dans les exemples est de type gène suicide.

Le trangène peut également être une cytokine ou une molécule d'intérêt thérapeutique.

L'expression "séquence pseudo-rétrovirale recombinante" signifie que la séquence comporte l'ensemble des gènes nécessaires à l'expression d'une particule pseudo-rétrovirale à l'exception du gène enveloppe (env) et comprenant éventuellement un ou plusieurs transgènes.

L'expression "cellule hôte" signifie la cellule contenant les deux séquences d'acides nucléiques recombinants, portées ou non par le même plasmide, et utilisable soit comme médicament, soit pour produire les virus recombinants défectifs.

L'expression "cellule cible" signifie la cellule que l'on cherche à traiter par introduction du transgène.

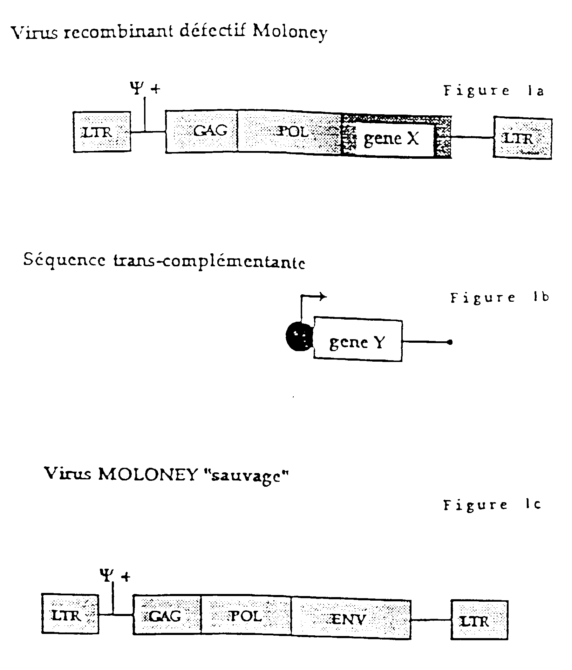

La présente invention répond et permet de surmonter les limites des systèmes existants citées ci-dessus par la construction d'un système hôte-vecteur permettant d'exprimer un transgène dans une cellule cible ou un tissu humain ou animal, caractérisé en ce qu'il est constitué d'une cellule eucaryote établie en lignée, dans laquelle ont été transfectées :
a) une séquence pseudo-rétrovirale recombinante dans laquelle le gène env a été délété dans sa totalité ou partiellement et substitué par ledit transgène au niveau du gène env tel que représenté sur la figure la, de sorte que le transgène soit exprimé sur des transcrits épissés similaires à ceux du gène env dans le virus Moloney sauvage;
b) une séquence d'acides nucléiques incluant une séquence codant pour une protéine d'enveloppe ou membranaire, laquelle séquence est sous la dépendance d'un promoteur et est associée le cas échéant audit transgène, et flanquée à son extrémité 3' un site de polyadénylation et représenté sur la figure 1b ; ledit génome viral recombinant et ladite séquence étant susceptibles de se complémenter en trans et permettre à ladite cellule hôte de produire des virus infectieux défectifs dépourvus du gène env.

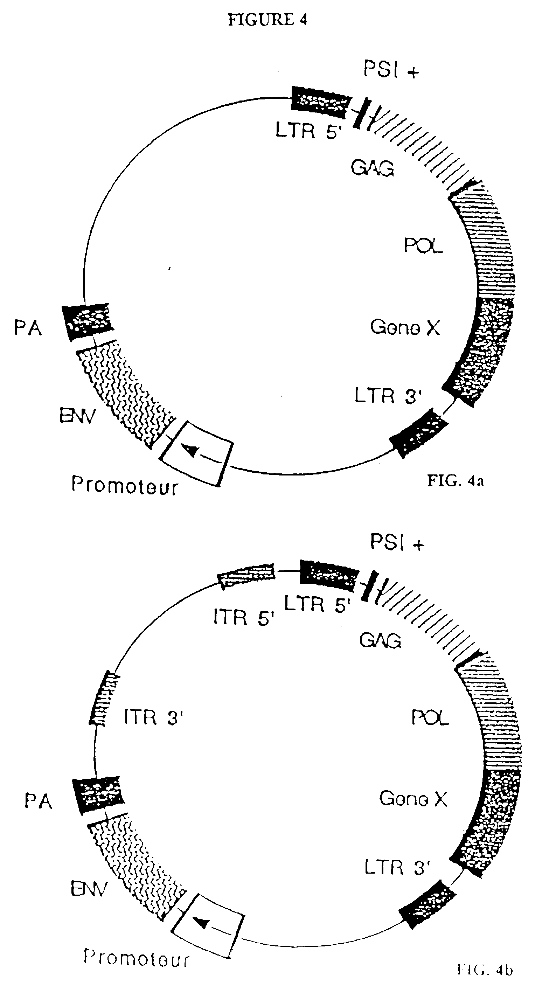

Le génome viral recombinant décrit en a) et la séquence d'acides nucléiques portant le gène env. décrite en b) peuvent être portés soit par deux plasmides ou systèmes de transfection differents, soit par le même plasmide ou système de transfection pourvu que la séquence b) soit située en dehors de la séquence pseudo-rétrovirale comprise entre les deux LTR. Une construction sur un seul plasmide est représentée sur la figure 4a.

La caractéristique commune aux deux types d'approches : deux supports distincts ou un seul support pour les séquences a) et b) est que la séquence b) étant dépourvue de la séquence ψ, elle n'est jamais empaquetée et par conséquent les virus produits par l'hôte en sont dépourvus.

En outre, dans ce dernier cas, le plasmide portant les deux séquences a) et b) ou celui portant la séquence pseudo-rétrovirale de la figure la ci-dessus peuvent porter d'autres séquences recombinantes destinées à améliorer la stabilité ou la durée de l'expression desdites séquences. Ces éléments complémentaires peuvent par exemple être les ITR (pour : Inverted Terminal Repeat) du virus AAV (Adeno Associated Virus). Un exemple d'une telle construction est représentée sur la figure 4b.

Il apparaît clairement dans ce système que la cellule hôte abritant les deux séquences recombinantes ci-dessus portées par un ou deux plasmides ne peut être assimilée à une cellule d'empaquetage dans la mesure où la première séquence pseudo-rétrovirale recombinante est porteuse de la séquence psi et permet, lorsqu'elle est transfectée seule, indépendamment de la deuxième séquence contenant le gène env, la production par la cellule hôte de particules virales porteuses de l'ensemble de la séquence de départ, mais qui sont dépourvues d'enveloppe et donc non infectieuses.

La cellule se comporte donc bien comme l'hôte d'un vecteur viral normal ou recombinant autorisant la production de particules virales sans adjonction de Helper ou de co-expression en trans comme décrit précédemment.

L'addition dans cette cellule hôte d'un gène codant pour la protéine d'enveloppe virale sous la dépendance d'un promoteur et suivie d'une séquence de polyadénylation permet la transcription, puis la traduction de ce gène en protéine dans la cellule hôte et, de façon subséquente, la reconstitution de particules virales porteuses du même génome que les précédentes (et représentée sur la figure 1a) à ceci près qu'elles possèdent désormais une enveloppe qui leur permet d'infecter des cellules cibles. Ces particules virales infectieuses ne sont, après infection d'une cellule cible, capables de produire des particules virales infectieuses que si le gène env est ajouté à nouveau et est exprime.

Les plasmides recombinants de l'invention qui ont comme caractéristiques de posséder la séquence rétrovirale recombinante encadrée des deux LTR, et un gène env. porté ou non par le même support plasmidique, peuvent être également associés à un système permettant leur introduction dans les cellules cibles. Ces systèmes peuvent être de plusieurs types tels des virus ou un véhicule approprié favorisant le transfert du matériel génétique à l'intérieur des cellules ; les véhicules appropriés sont définis comme permettant le passage des membranes biologiques et peuvent être notamment des liposomes, des lipides cationiques, des dérivés de polylysine, des adénovirus inactivés ou des méthodes balistiques.

Des liposomes ont été utilisés pour envelopper et transférer dans des cellules aussi bien des acides nucléiques que des particules virales (R. Philips et al, (1994) Molecular and Cellular Biol. Vol 14 n° 4, p 2411-2418). Ces véhicules présentent une piètre efficacité de transfection des plasmides qu'ils contiennent. Le fait d'adjoindre à la séquence pseudo-rétrovirale de l'invention des séquences permettant une expression meilleure et prolongée des séquences d'intérêt thérapeutique permet d'envisager leur utilisation comme (trans)porteurs de plasmides recombinants soit comme moyen intermédiaire à la fabrication d'un médicament, soit comme principe actif de médicament ; la cellule ainsi transfectée par le véhicule contenant les vecteurs se transforme alors elle-même en cellule productrice de rétrovirus défectifs codant pour le gène d'intérêt mais dépourvus du gène enveloppe et le virus défectif est lui-même capable de réinfecter d'autres cellules pour y exprimer à nouveau le gène d'intérêt, le cycle s'arrêtant nécessairement à ce stade, sauf ajout d'une séquence env. par quelque moyen que ce soit. Cette cellule est soit la cellule du système hôte vecteur soit la cellule cible telle que définies plus haut.

L'invention est relative également aux véhicules tels que définis plus haut contenant une séquence pseudo-rétrovirale telle que décrite en la, une séquence recombinante portant un gène env. telle que décrite en 1b, séparés ou rassemblés sur un même support plasmidique tel que décrit dans les figures 4a et 4b, et portant en option des ITR du virus AAV en amont et en aval de la séquence recombinante pseudo-rétrovirale, et de préférence en aval de la séquence recombinante portant le gène env.

De manière plus générale, tout système de transfection dans lequel :
- un vecteur viral recombinant contenant un trangène d'intérêt, dans lequel un gène essentiel à la constitution de particules virales infectieuses est substitué par ledit transgène d'intérêt,
- ce gène essentiel est présent sur le même vecteur, ou un vecteur distinct, mais n'est plus sous la dépendance des promoteurs viraux et,
- le produit de ce gène agit en trans et permet de reconstituer des particules virales défectives, fait partie de l'invention.

Un autre exemple de construction de ce type, non rétrovirale, est la reconstitution d'un adénovirus recombinant défectif dans lequel le gène E1A est substitué par un transgène d'intérêt, et la séquence E1A est exprimée en trans de telle façon à reconstituer une particule virale infectieuse défective.

La caractéristique essentielle des constructions de l'invention, c'est qu'elles permettent de produire des virus infectieux soit dans une cellule hôte, soit dans une cellule cible, mais les virus produits contiendront un acide nucléique capable d'exprimer un transgène et incapable de produire des particules infectieuses.

Dans le cas de particules pseudo-virales décrites plus haut, la séquence env. sera en dehors de la zone de régulation de l'expression des séquences rétrovirales.

Ce système est particulièrement avantageux comme nous le verrons par la suite lorsque le transgène à exprimer est un gène suicide, notamment la thymidine kinase du virus Herpès Simplex (HSV1-TK), ou une cytokine.

Dans le système hôte-vecteur de l'invention, la séquence pseudo-rétrovirale recombinante est dérivée du génome du virus de Moloney MuLV, les séquences LTR en 5' ou 3' étant d'origine sauvage ou dérivées de différents mutants ou combinées. On pourra citer par exemple les constructions de type LTR dérivées des mutants mov3, mov9 et mov13 tels que décrits dans Caruso M. et al. dans Proc. Natl. Acad. Sci. USA, 90:7024-7028, 1993.

Cette séquence d'acides nucléiques (figure 1a), qui va être encapsidée dans la particule virale produite par la cellule hôte, est extrêmement proche du génome rétroviral "sauvage", ce qui présente l'avantage d'obtenir des titres plus élevés que ceux obtenus dans les systèmes décrits ci-dessus dans l'état de la technique avec les lignées de packaging et les complémentations en trans des gènes viraux.

A titre d'exemple, et comme nous le verrons plus loin, alors que dans les systèmes de l'état de la technique le titre maximum avec les cellules de packaging est de l'ordre de 106, ce système permet des titre compris entre 106 et 109 et de préférence entre 107 et 108 PFU/ml.

Une deuxième caractéristique du système hôte-vecteur de l'invention est que la cellule hôte est transfectée simultanément avec une séquence d'acides nucléiques incluant une séquence codant pour une protéine d'enveloppe, cette protéine étant d'origine virale, voire rétrovirale, ou protéine membranaire, voire cellulaire.

"Simultanément" signifie que les deux séquencés, la séquence pseudo-rétrovirale et la séquence d'acides nucléiques codant pour le gène enveloppe, sont exprimées simultanément dans la cellule hôte, les transfections pouvant être réalisées soit simultanément sur une ou deux constructions, soit successivement.

Le gène enveloppe choisi peut être homologue à la séquence pseudo-rétrovirale, c'est-à-dire être issu par exemple du Mo-MuLV, permettant la reconstitution des particules pseudo-virales homologues ou être issu d'un autre virus, par exemple et sans être limitatif le virus de la stomatite vésiculaire (VSV), le HIV, le virus de la rage ou le virus de la leucémie du gibbon, lequel gène lorsqu'il est transcrit et traduit en protéine d'enveloppe permet la reconstitution d'un pseudo-type viral dont l'enveloppe est une enveloppe de VSV (ou de HIV) et le génome comportant les LTR, psi, PB, gag et pol du MuLV, ainsi que le transgène.

Enfin, le génome env peut être d'origine cellulaire et coder pour une protéine membranaire permettant le ciblage de la particule virale sur un ligand spécifique, rotamment pour un récepteur de type CD4.

En outre, l'enveloppe peut être une protéine chimérique dont l'extrémité carboxyterminale est dérivée des séquences intramembranaires de l'enveloppe du Moloney. Il existe en effet des données expérimentales qui montrent que ces séquences membranaires peuvent être impliquées dans la concentration de ces protéines d'enveloppe à la surface de la particule virale. Avec ces molécules chimériques, l'efficacité d'expression de la protéine env chimérique à la surface des virions sera accrue.

Un deuxième avantage de ce système hôte-vecteur permettant l'expression de ces pseudo-types viraux hétérologues est la possibilité éventuelle de purifier ces pseudo-particules virales par ultracentrifugation sans perdre de pouvoir infectieux. Ceci permet d'obtenir des suspensions virales dont le titre peut atteindre 109 PFU/ml.

La séquence d'acides nucléiques contenant le gène env tel que décrit dans la figure 1b ci-après, peut être transfectée par tout type de moyens, que ce soient des moyens physiques ou à l'aide de vecteurs viraux ou rétroviraux. Parmi les moyens physiques connus, on pourra citer la micro-injection, les liposomes ou encore des procédés dits de biolistique ou par bombardement tels que décrits dans Kriegler, M. Gene Transfer and Expression; A Laboratory Manual; MacMillan Publishers Ltd.; 1990.

La transfection peut également avoir lieu par intégration de cette séquence d'acides nucléiques dans un vecteur viral et notamment de l'adénovirus.

L'avantage de cette dernière méthode est la possibilité de mettre en place un système auto-entretenu in vivo tout en gardant l'entière maîtrise de la prolifération virale.

En effet, le système hôte-vecteur comprenant comme transgène un gène suicide et injecté par exemple au niveau d'une tumeur va exprimer une particule virale recombinante qui, elle-même, va réinfecter in situ des cellules en division.

Ces cellules pourront à leur tour réexprimer une particule virale, mais, cette dernière sera non infectieuse puisque le génome viral n'aura pas été complémenté en trans par un gène d'enveloppe.

Si, à cette étape, il est procédé à une injection de vecteur d'expression recombinant contenant le gène env, il pourra y avoir à nouveau trans-complémentation et le cycle reproduit une fois de plus ; la sécurité du système est, de cette façon, présérvée puisqu 'une double transfection est nécessaire, l'une par la pseudo-particule virale MuLV susceptible d'infecter uniquement les cellules en division et l'autre par l'adénovirus susceptible d'infecter l'ensemble des cellules. Donc, seules les cellules en division seront susceptibles d'être co-transfectées, d'exprimer les gènes viraux en trans et de produire des pseudo-particules virales susceptible d'infecter les cellules cibles.

Ce système permet donc de façon particulièrement élégante de transfecter dans une cellule cible, par exemple le gène suicide TK, permettant l'expression de ce dernier et conférant à la cellule qui le contient la sensibilité aux analogues de nucléosides ; la transfection simultanée d'un gène enveloppe permet la production par cette même cellule cible de particules infectieuses comprenant le même génome pseudo-viral recombinant défectif en gène enveloppe.

Ce cycle peut donc être réitéré le nombre de fois désiré tant que la séquence porteuse du gène env est apportée de façon exogène ; le système s'arrête après un cycle réplicatif du virus dans la cellule cible, production de particules infectieuses, réinfection et production de particules non infectieuses.

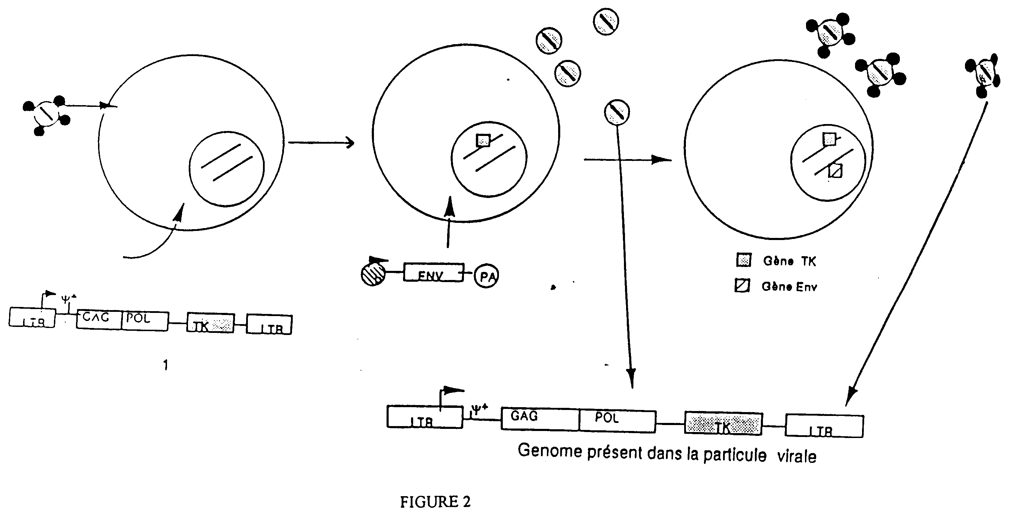

L'ensemble de ce cycle est décrit dans la figure 2.

Un troisième avantage du système hôte-vecteur selon l'invention est que tout type de cellules eucaryotes établies en lignées peut être choisi à partir du moment où cette lignée est susceptible de produire ce type de particules virales amphotropes.

Outre les lignées NIH/3T3 utilisées classiquement pour produire des particules virales recombinantes du virus de Moloney, le système hôte-vecteur de l'invention autorise l'utilisation de lignées cultivables en suspension ; à titre d'exemple, on peut citer les myélomes de souris, les cellules VERO ou les cellules d'insectes.

L'avantage évident d'utiliser des cellules en suspension est l'application industrielle pour la préparation de médicaments utilisables en thérapie génique, des quantités importantes de ces cellules étant nécessaires à de telles préparations qui sont bien évidemment beaucoup plus aisées d'obtention avec des cellules en suspension qu'avec des cellules adhérentes.

Le choix des cellules VERO comme cellules hôtes peut présenter l'avantage d'une part de provenir de primates et donc être mieux adaptées phylogénétiquement à l'homme et d'autre part d'être plus résistantes à l'action des anticorps naturels et du complement. Ces cellules ont enfin été utilisées largement pour la production de vaccins, et en particulier de virus qui ne subissent que très peu d'étapes d'inactivation ou de purification (vaccin contre la Rage par exemple). Certaines cellules utilisables à titre de cellules hôtes productrices de particules virales ont la propriété que lesdites particules ne sont pas inactivées par le complément : il s'agit notamment des lignées cellulaires humaines ou issues du vison.

Le choix comme cellules hôtes de cellules des lépidoptères ou plus généralement des cellules d'insectes (à l'exception des diptères) présente également de nombreux avantages pour construire des systèmes hôte-vecteur utilisables à titre de médicaments en thérapie génique:
- elles sont capables de produire des protéines d'enveloppe virales de virus infectant les eucaryotes, mais elles-mêmes ne sont pas infectables par ces virus ;
- les cellules d'insectes ne produisent pas de virus de mammifères, les tests de sécurité virale les concernant sont donc réduits ;
   ces cellules ne sont pas infectables par des virus potentiellement pathogènes pour l'homme ; il est donc beaucoup plus sûr d'utiliser ces cellules pour un usage en thérapeutique humaine sur le plan des tests de sécurité virale ;
- les cellules d'insectes poussent en suspension et donc sont cultivables à grande échelle ;
   les cellules d'insectes sont capables de déplacements intratissulaires ; aussi, dans le cas où elles seraient injectées au sein d'une tumeur, elles peuvent donc s'y mouvoir et délivrer leurs particules pseudo-virales à distance du lieu d'injection ;
- il est possible d'utiliser des promoteurs spécifiques aux cellules d'insectes qui sont des promoteurs forts : par exemple, il est possible d'utiliser des promoteurs de baculo virus qui donnent de forts taux d'expression et donc un nombreux accru de particules pseudo-virales ; étant donné qu'il y a plusieurs promoteurs forts issus de baculo virus, il est donc possible d'effectuer des constructions complexes afin de limiter les risques de recombinaison entre les différents gènes transfectés.

Tous ces avantages font que les cellules d'insectes, éventuellement modifiées génétiquement afin d'être capables de fabriquer des particules pseudo-virales amphotropes sont un outil de choix pour la construction du système hôte-vecteur de l'invention.

L'homme du métier saura, au cas par cas, selon le transgène qu'il souhaite exprimer dans une cellule cible, réaliser les constructions en utilisant les vecteurs d'expression appropriés et les cellules hôtes appropriées permettant la transcomplémentation du gène enveloppe avec le génome rétroviral porteur de la séquence psi, des gènes gag, pol et du ou des transgènes, et permettant une dissémination par l'adjonction du gène env, contrôlée dans le temps et dans l'espace.

Un autre perfectionnement avantageux du système hôte-vecteur de l'invention consiste à intégrer, dans la séquence d'acides nucléiques porteuse du gène env, une séquence du gène de la thymidine kinase de HSV1 ou tout autre gène suicide conditionnel ; ce perfectionnement est une deuxième clé permettant d'augmenter l'efficacité du traitement, ainsi que la sécurité du système, puisque, de cette manière, le gène suicide est exprimé dans la cellule cible sous la dépendance de promoteurs de types différents et, par conséquent, cela permet d'augmenter la sensibilité aux analogues de nucléosides conférée aux cellules par l'expression de ce gène suicide.

Un autre perfectionnement particulièrement avantageux pour des problèmes de sécurité virale dans une utilisation thérapeutique chez l'homme est l'utilisation d'un système hôte-vecteur dans lequel :
- les cellules hôtes sont des cellules d'insectes, non productrices de virus de mammifères ;
- le vecteur portant le gène env utilisé sous la dépendance d'un promoteur comporte en amont du gène env une séquence homologue du rétrovirus recombinant défectif utilisé dans le système hôte-vecteur, par exemple tout ou partie d'un gène gag ou tout ou partie d'un gène pol.

Ce système est intéressant car, si une recombinaison homologue avec un rétrovirus se produit dans un système où l'hôte comporte des rétrovirus endogènes, par exemple dans des cellules de souris, alors elle conduira à la production de rétrovirus non fonctionnels. Cette recombinaison aura lieu de telle sorte que le génome recombinant qui en résulte sera également défectif. L'injection de ce type de cellules à l'homme sera de toute sécurité sur le plan viral.

De même, l'utilisation avec ce système d'une cellule d'insecte dans le système hôte-vecteur ne peut conduire à des recombinaisons homologues, les cellules d'insectes ne portant pas de rétrovirus de mammifères et, par conséquent, l'injection à des fins thérapeutiques ne pourra permettre de produire que les particules virales recombinantes défectives de l'invention, à l'exclusion de particules virales issues d'une recombinaison homologue et qui pourrait alors être infectieuse.

La présente invention est également relative à un procédé d'expression d'un transgène pour la thérapie génique qui consiste en la mise en oeuvre des étapes suivantes :
a) construction d'un système hôte-vecteur par transfection dans une cellule eucaryote hôte, d'une part, d'une séquence pseudo-rétrovirale dans laquelle le gène env est délété dans sa totalité et remplacé par le transgène, par exemple au niveau de l'ATG dudit gène env et, d'autre part, une séquence d'acides nucléiques comportant dans sa structure une séquence codant pour une protéine d'enveloppe sous le contrôle d'un promoteur, le cas échéant associé au transgène et flanqué à son extrémité 3' d'une séquence de polyadénylation, les deux séquences ci-dessus étant portées par un ou deux vecteurs de type plasmidique,
b) mise en contact dudit système avec les cellules dans lesquelles le transgène doit être exprimé,
c) le cas échéant, transfert a nouveau dans les cellules cibles de la susdite séquence contenant le gène env. Dans le cas où les deux séquences sont portées par la même construction, la séquence comportant la séquence codant pour une protéine env. est extérieure aux deux LTR 5' et 3' de la séquence pseudo-rétrovirale.

Dans le procédé de l'invention, le transgène peut être un gène d'intérêt thérapeutique et notamment un gène suicide tel le gène de la thymidine kinase dé HSV1 conférant, comme il a été dit plus haut, la sensibilité des cellules exprimant ce gène à des analogues de nucléosides.

Dans le procédé de l'invention, la séquence pseudo-rétrovirale est directement dérivée du virus de Moloney MuLV, les séquences LTR en 5' ou en 3' étant dérivées directement d'espèces virales particulières telles le mov9, mov3 ou mov13, elles peuvent être d'origine sauvage, mutante ou combinées.

Dans le procédé de l'invention, la séquence contenant le gène env est choisie notamment parmi les séquences de rétrovirus codant pour ledit gène et notamment le gène env du MuLV ou le gène env d'un virus hétérologue tel par exemple le VSV, le HIV, le virus de la rage, le virus de la leucémie du Gibbon. Il va de soi que la particule virale reconstituée est une particule hybride dont l'enveloppe est constituée de la protéine d'enveloppe du virus dont a été issue la séquence env et le génome viral étant issu du MuLV.

Le procédé selon l'invention est caractérisé par le fait que la séquence contenant le gène env (que cette séquence soit incluse dans un plasmide portant également la séquence pseudo-rétrovirale ou au contraire qu'elle soit incluse dans un vecteur autonome de cette dernière) est introduite dans la cellule cible par tout moyen approprié, notamment par un vecteur viral, tel l'adénovirus, ou encore par des méthodes physiques, par exemple bombardement, fusion de liposomes ou micro-injection. Il va de soi, comme cela a été expliqué plus haut, que l'utilisation, d'un adenovirus permet la mise en oeuvre d'un procédé à haut rendement et tout en conservant une sécurité totale puisque l'expression de particules virales ne peut être réalisée que dans les cellules en division et par transcomplémentation de la pseudo-particule virale derivée du virus de Moloney et du gène enveloppe dérivé du vecteur adénoviral.

Une variante avantageuse du procédé de l'invention, notamment quand les liposomes sont utilisés comme moyens de transfection, est l'addition d'une ou plusieurs séquences, à l'extérieur des deux LTR, dont la fonction serait une fonction stabilisatrice de l'expression de la séquence pseudo-rétrovirale et notamment du transgène.

Un exemple de ces séquences est les séquences ITR (Invented Terminal Repeat) du virus AAV. Ces séquences sont susceptibles, quand elles sont présentes de part et d'autre de séquences à exprimer, et notamment en amont, de conférer une stabilisation substantielle de l'expression desdites séquences (Philips et al, supra et Flotte T.T. et al. Am. J. Resp. Cell. Mol. Biol, 1992 7: 349).

Hors ce type de séquences, tout type de séquence susceptible d'augmenter l'expression en terme de taux et/ou de durée peut être choisie par l'homme du métier et intégrée dans le vecteur ou plasmide de transfection à l'extérieur des deux séquences nucléosidiques de type a) et b) ci-dessus.

De façon plus générale, l'invention est relative à un procédé permettant l'expression d'un transgène pour la thérapie génique caractérisé par la transfection simultanée dans des cellules cibles d'un génome rétroviral de structure générale représentée dans la figure la, lequel génome possède toutes les séquences nécessaires à l'expression d'une particule virale, mais est dépourvu du gène enveloppe conférant à ladite particule virale son caractère infectieux et, d'autre part, une séquence d'acides nucléiques contenant dans sa structure un gène enveloppe de virus ou cellulaire, la co-expression en trans des deux séquences ainsi transfectées permettant l'expression de particules virales recombinantes infectieuses mais dépourvues dans leur génome dudit gène env.

Cette co-expression en trans des deux types de séquences d'acides nucléiques peut être réalisée soit dans une cellule hôte permettant donc la réalisation d'un système hôte-vecteur utilisable en tant que tel en thérapie génique, soit en injection simultanée au niveau des cellules cibles à traiter, soit enfin par encapsulation dans un véhicule, par exemple des liposomes.

Cette expression peut être réalisée aussi bien quand les séquences sont portées par deux structures différentes, soit par la même structure plasmidique pourvu que celle portant le gène env. soit extérieure aux deux LTR 5' et 3'de la séquence pseudo-rétrovirale.

Il apparaît clairement à l'homme du métier le caractère itératif du système utilisé par adjonction en tant que de besoin de la deuxième séquence d'acides nucléiques porteuse du gène env et susceptible d'être exprimée sous le contrôle d'un promoteur.

L'invention est également relative a l'utilisation en thérapie génique du système hôte-vecteur décrit plus haut et obtenu par transfection simultanée dans une cellule eucaryote de deux séquences d'acides nucléiques dont l'expression en trans permet à ladite cellule eucaryote de produire des particules virales infectieuses et porteuses du transgène, cette utilisation pouvant être avantageusement appliquée à l'expression d'un gène suicide dans des cellules cibles à détruire.

Cette co-expression est réalisée par une double transfection de séquences décrites dans les figures 1a et 1b, ou par transfection d'une structure unique portant les deux types de séquences 1a et 1b, dont un exemple est représenté dans la figure 4a.

Si cette structure unique porte en outre une séquence améliorant l'expression en terme de taux ou de durée, telles les séquences ITR du virus AAV, l'utilisation est particulièrement avantageuse, notamment dans le cas de l'utilisation des liposomes comme moyen de transfection, améliorant ainsi l'efficacité de cette technique et permettant l'utilisation des liposomes porteurs des plasmides recombinants comme intermédiaire dans la fabrication d'un système hôte-vecteur utilisable en thérapie génique ou aussi comme principe actif de médicament utilisable en thérapie génique.

Les ITR d'AAV produisent leur effet maximal quand ils sont positionnés pour l'un en amont du LTR5' et pour l'autre en aval de la séquence de polyadénylation PA, telle que représentée dans la figure 4b.

Enfin, l'invention est relative à des médicaments utilisables en thérapie génique et caractérisés en ce qu'ils contiennent comme principe actif des cellules eucaryotes ayant subi une double transfection par les séquences d'acides nucléiques telle que décrite dans les figures 1a et 1b ; le principe actif d'un médicament selon la présente invention peut être également constitué, d'une part, de particules virales recombinantes infectieuses porteuses d'un génome viral dans lequel le gène env a été substitué par exemple au niveau de son codon d'initiation par le transgène à exprimer et, d'autre part, par une séquence d'acides nucléiques comportant dans sa structure le gène enveloppe, sous la dépendance d'un promoteur et, le cas échéant, flanquée par une séquence codant par exemple pour le gène de la thymidine kinase.

L'invention est de la même façon relative à des médicaments caractérisés en ce qu'ils contiennent comme principe actif des liposomes contenant des séquences d'ADN recombinantes dont l'une est constituée d'une séquence pseudo-rétrovirale comprise entre des LTR 5' et 3' de rétrovirus, comprenant les gènes gag et pol et un gène d'intérêt thérapeutique, mais dépourvu du gène env., et une autre séquence extérieure a la première comportant un gène env. et une séquence de polyadénylation et sous la dépendance d'un promoteur et le cas échéant également flanqué par une séquence codant pour un gène d'intérêt ; dans un mode de realisation avantageux, le médicament constitué de ces liposomes porteurs des séquences recombinantes est tel que le plasmide qui les porte comprend en outre des séquences permettant d'améliorer l'expression des gènes d'intérêt et notamment ceux qui sont compris entre les deux LTR augmentant ainsi l'efficacité dudit médicament.

Le gène d'intérêt des médicaments de l'invention est de manière avantageuse le gène de la thymidine kinase, qui, quand il est exprimé, confère à la cellule qui l'abrite une sensibilité aux analogues de nucléotides tels que le gancyclovir ou l'acyclovir.

L'homme du métier saura mettre en oeuvre toutes les variantes possibles de ce type de système hôte-vecteur, par expérimentation de routine.

De telles variantes, ou équivalents, font partie de l'invention telle que revendiquée ci-après.

Les figures et les exemples qui suivent permettront d'illustrer de façon plus précise l'invention.
- La figure 1 représente la construction des deux séquences d'acides nucléiques devant être transfectées simultanément.
   Dans cette figure :
   . la figure 1a représente le virus recombinant défectif Moloney dans lequel la zone en gris représente des séquences dérivées du virus de Moloney et le gène X sur fond noir représente le transgène ;
   . la figure 1b représente un gène Y codant pour une enveloppe virale ou pour une protéine membranaire flanqué en 5' d'un promoteur et éventuellement d'une séquence de rétrovirus, et flanqué en 3' d'une séquence de polyadénylation ;
   . la figure 1c, pour mémoire, indique la séquence du virus Moloney sauvage.
- La figure 2 représente le principe de la co-expression en trans et de la production de particules virales défectives et infectieuses permettant la réintroduction dans une cellule cible du génome rétroviral recombinant défectif.
   Dans la première partie du schéma, un vecteur de type représenté dans la figure la est introduit dans la cellule hôte. (Il est à noter que cette introduction peut être réalisée, soit par transfection, soit par infection par une particule rétrovirale défective telle que décrite à la fin de ce schéma). Le résultat de cette expression du vecteur représenté dans la figure la est la production de particules virales dont le génome est dérivé de ce vecteur mais qui ne portent pas à leur surface de protéine d'enveloppe et qui sont donc non infectieuses (telles que représentées dans la 2ème partie du schéma).
   On exprime alors par transfection dans cette cellule un vecteur du type représenté dans la figure 1b, porteur du gène env. La complémentation en trans permet alors l'expression à la surface des particules virales de la protéine env, ces particules virales restant dépourvues du gène env.

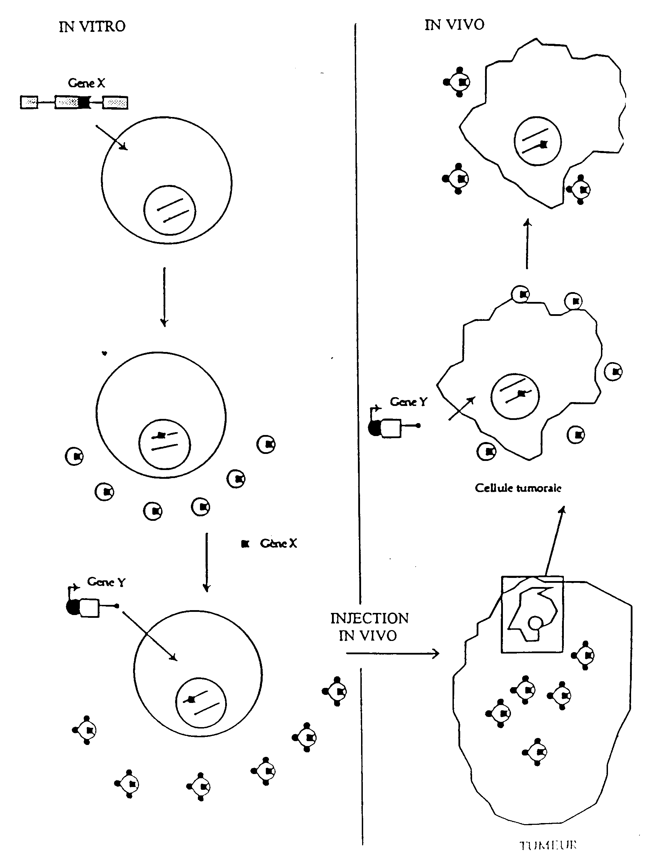

- La figure 3 représente le schéma thérapeutique depuis la génération des cellules produisant les particules virales infectieuses porteuses du transgène jusqu'à leur injection in situ dans la tumeur ainsi que la perpétuation du cycle infectieux par la complémentation in situ avec un gène d'enveloppe. La colonne de gauche reprend la production de particules virales recombinantes défectives telles que déjà représentées dans la figure 2, lesquelles cellules sont injectées in vivo dans une tumeur. Les particules virales recombinantes défectives produites in situ sont alors susceptibles d'infecter des cellules tumorales ; l'effet thérapeutique du transgène peut alors s'exprimer dans ces cellules et les cellules tumorales transduites produisent elles-mêmes des particules virales porteuses du transgène et depourvues d'enveloppe. Le cycle s'arrête à ce niveau, sauf ajout ultérieur du vecteur selon la figure 1b.

La figure 4a représente une construction plamsmidique dans laquelle les deux séquences -pseudo-rétrovirales et env. - sont portées par la même structure.

La figure 4b est une variante dans laquelle les ITR du virus de l'AAV ont été ajoutés en amont du 5' LTR et en aval de la séquence de polyademylation (PA).

Le transgêne est dans l'un et l'autre cas le gène de la thymidine kinase de HSV1.

La figure 5 représente un couple particulier de vecteurs utilisés pour montrer l'efficacité du système de l'invention, la figure 5a étant le vecteur pNP-2 contenant le gène HSV1-TK, et le plasmide p CRIP GAC-2 contenant le gène env du virus de Moloney, tels que décrits dans l'exemple 3 ci-après.

### - EXEMPLE I : Construction d'un virus de Moloney porteur d'un gène thérapeutique et défectif pour l'enveloppe virale :

Le vecteur utilisé est dérivé du vecteur rétroviral pMA 245, lequel est dérivé du virus de Moloney MuLV et a été construit à partir de fragments MuLV clonés à partir de différentes espèces de Mov (Mov-3, -9, et -13) telles qu'établies par Jaenisch et al., 1981, Cell 24:519-529.

La construction génétique est facilement réalisable par l'homme de métier. On part d'un plasmide porteur d'un provirus Moloney infectieux. Un tel plasmide, lorsqu'il est transfecté dans une lignée cellulaire adéquate (par exemple cellule NIH-3T3 de souris), donne naissance à des particules virales infectieuses porteuses du génome de Moloney sauvage dérivé de la construction génétique transfectée. Dans ce génome, on substitue le gène codant pour l'enveloppe par un gène codant pour la protéine d'intérêt thérapeutique. Cette construction est réalisée de sorte que ce gène est synthétisé à partir des mêmes transcrits épissés que le gène d'enveloppe "sauvage". Dans un souci d'efficacité de l'expression du gène d'intérêt thérapeutique, et également d'efficacité de la production de particules rétrovirales, on peut même substituer le gène d'intérêt thérapeutique de sorte que le codon ATG de celui-ci se trouve exactement à la position de l'ATG du gène env de Moloney. Le gène d'intérêt thérapeutique est plus volontiers dérivé de l'ADN complémentaire et ne comporte donc pas d'intron et est également dépourvu de séquence de polyadénylation. La construction génétique est telle qu'elle respecte donc l'épissage naturel du virus de Moloney, qu'elle respecte les cadre de lecture des gênes gag et pol et qu'elle respecte l'intégrité du LTR3' du virus de Moloney. L'ensemble de la construction génétique est réalisé selon les règles de l'art par digestion enzymatique de l'ADN puis ligation des fragments appropriés ; ces techniques sont décrites dans Maniatis et al., 1989, Molecular Cloning, A Laboratory Manual. Si nécessaire, elle utilise la PCR ou toute technique d'amplification appropriée pour générer des fragments génétiques aux extrémités desquels se trouvent les sites de restriction appropriés au clonage.

Si nécessaire également, les différents fragments génétiques composant le provirus infectieux Moloney peuvent être décomposés en fragments plus courts répartis sur les plasmides distincts, de sorte qu'ils soient manipulés plus simplement pour être ensuite réassemblés de façon appropriée. Si nécessaire enfin, on peut utiliser la mutagénése dirigée pour introduire, soit des sites de restriction nécessaires à la réalisation de la construction génétique, soit pour substituer à des positions précises les différents gènes. A l'issue de cette construction génétique, on dispose d'un plasmide qui, lorsqu' il est transfecté dans une cellule (par exemple NIH-3T3), s'exprime comme un virus de Moloney sauvage et aboutit donc à la génération de particules virales dont le génome est constitué par le provirus lui-même. Du fait de l'homologie entre le provirus infectieux et le provirus défectif nouvellement construit, la synthèse des différents ARN se fait avec une efficacité dans des proportions optimisées qui font que le nombre de particules virales qui sont fabriquées par la cellule est très voisin de celui d'un virus sauvage. Ce nombre de particules virales est donc largement supérieur à celui fabriqué par des lignées d'encapsidation classique dans lesquelles les différents gènes de structure du virus de Moloney sont portés par des constructions génétiques différentes, et dans laquelle le vecteur rétroviral qui constitue le génome de la particule virale est sur un plasmide différent et a une structure et une taille très différentes de celles du Moloney sauvage. Il est à noter que les particules virales ainsi produites ne sont pas infectieuses car elles sont dépourvues d'enveloppe. Cette protéine d'enveloppe peut être apportée de façon indépendante dans cette cellule grâce à un vecteur dont la construction est effectuée ci-après.

### - EXEMPLE II : Construction de la séquence d'acides nucléiques porteuse du gène env :

On peut en effet transfecter dans cette cellule une nouvelle construction génétique simple contenant un gène à expression membranaire sous le contrôle d'un promoteur classique (comme par exemple le promoteur du virus SV40, un promoteur de cytomégalovirus, un promoteur d'un gène housekeeping comme le PGK, ou bien même un promoteur spécifique d'un type cellulaire donné et qui offrira donc une sécurité d'emploi supplémentaire). Ce vecteur d'expression comprend également des séquences de polyadénylation provenant de différentes constructions génétiques (séquences de polyadénylation de SV40, de βglobine, de l'hormone de croissance...). Le gène membranaire peut être soit une enveloppe du virus Moloney lui-même, soit une enveloppe d'autre virus qui puisse être captée par la particule virale et exprimée à la surface de celle-ci, soit même une protéine cellulaire membranaire qui puisse également être présente à la surface de la particule virale. C'est le cas notamment de la molécule CD4 dont il a été montré qu'elle pouvait être incorporée par différents rétrovirus.

Un autre mode de réalisation est la construction des protéines membranaires chimériques, telles que leur partie intracellulaire est dérivée de la partie intracellulaire de l'enveloppe du virus Moloney, et leur partie extracellulaire est une protéine membranaire permettant de mieux cibler la particule virale. La présence des régions intracellulaires de l'enveloppe du Moloney assure probablement une meilleure efficacité d'expression de cette enveloppe chimérique à la surface de la particule virale.

Cette deuxième construction génétique est également réalisée de sorte qu'elle ne contienne aucune séquence dérivée d'un virus de Moloney autre que le gène enveloppe lui-même. Plus précisément, il ne peut contenir de séquence qui n'ait pas au préalable été délétée sur la construction génétique précédente. De la sorte, on minimise fortement la possibilité d'une recombinaison entre ce vecteur d'expression et le rétrovirus défectif tel qu'il a été décrit précédemment, recombinaison qui pourrait aboutir à la production d'un rétrovirus de Moloney infectieux cette fois-ci. Ces constructions génétiques sont également réalisées selon les règles de l'art comme indiqué précédemment.

### - EXEMPLE III : Expression de particules virales par transcomplémentation des deux séquences construites respectivement dans les exemples I et II :

Les constructions sont utilisées pour infecter des cellules de myélomes p3X63Ag8 ou des cellules 3T3 TK⁻déficientes en thymidine kinase.

L'expérience ci-dessous comprend deux expériences contrôle et deux essais :
lot a) : un premier contrôle de milieu de sélection, dans lequel les cellules ne sont pas transfectées,
lot b) : un deuxième contrôle de l'efficacité de la transfection dans lequel les cellules sont transfectées avec un plasmide contenant un gène codant pour la βgalactosidase (Lac Z)
lot c) : un essai dans lequel les cellules sont transfectées par le plasmide pNP-2 seul mais qui permet l'expression de TK mais pas la propagation via la fabrication de particules rétrovirales recombinantes défectives,
lot d) : un essai dans lequel les cellules sont co-transfectées par le vecteur pNP-2 et un plasmide porteur du gène env du virus de Moloney Mo-MuLV.

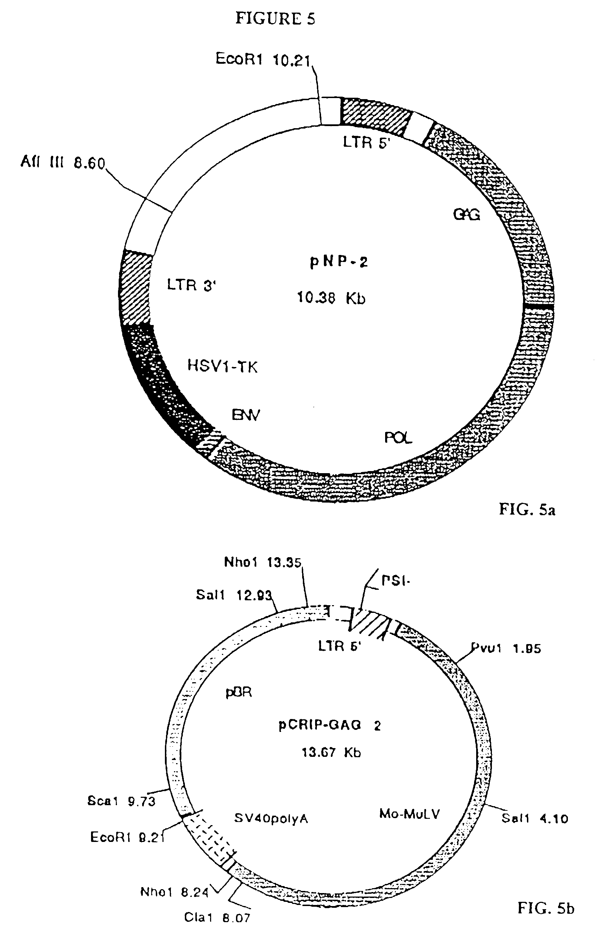

III.1 Description des plasmides
   - La figure 5a représente le plamside pNP-2 qui a été déposé à la CNCM le 20 février 1995 sous le n°I-1541. Il contient les gènes GAG et POL de Mo-MuLV, avec en aval un gène HSV1-TK sous un ATG de gène env muté. Sa taille totale est 10.38 KB. Les sites AF III (à 8.60 Kb) et ECoR1 (à 10.21 KB) sont figurés.
   - la Figure 5b représente le deuxième plasmide pCRIP GAG⁻², qui est un exemple de construction dans lequel le gène env du MoMuLV est intégré dans une séquence comportant les séquences MoMuLV, ψ- gag-, pol+, env+, et suivie en aval par une séquence polyA de SV 40.
      Les chiffres entre parenthèse indiquent la distance en nombre de paires de base.
III.2 Transfection
   Des cellules 3T3 TK sont soit non transfectées (lot a)), soit transfectées selon les conditions décrites plus haut (lots b), c) et d)).
   Dans le lot b), l'efficacité de transfection est révélée par utilisation de substrat chromogène ; pour cela, les cellules sont cultivées en présence d'IPTG (induction de l'opéron Lac) et au bout de 24h en présence de x gal dont la couleur passe au bleu quand il est clivé par la βgalactosidase exprimée. La présence de la couleur bleue dans les cellules révèle donc l'existence de la transfection.
   Dans les lots c) et d), les cellules sont mises en présence du milieu de sélection HAT soit à 24h, soit après 5 jours de culture où l'on s'attend à avoir un nombre de cellules résistantes sous HAT largement supérieur, compte tenu de la propagation cellulaire.
III.3 Résultats
   1) Efficacité de transfection : dans les conditions utilisées, l'efficacité de transfection avec le plasmide lac Z montre des valeurs aux alentours de 5% de cellules transfectées.
   2) Clones obtenus après une sélection dès la 24ème heure. Pour les transfections avec le plasmide pNP-2 seul, on voit moins d'une dizaine de clones de toute petite taille (entre 20 à 50 cellules par clone). Pour la transfection avec pNP-2 plus le gêne codant pour l'enveloppe de Moloney, on voit également une dizaine de clones de petite taille plus 38 clones de taille largement supérieure (>200 cellules par clone).
   3) Clones obtenus après une sélection HAT au 5ème jour. Pour les transfections avec pNP-2 seul, on retrouve quelques clones également de petite taille. Pour la boîte correspondant a la transfection de pNP-2 et le plasmide env, on observe un nombre de clones très important, incomptable, correspondant environ au quart de confluence sur l'ensemble de la boîte.
III.4 Conclusions
   1) Le plasmide pNP-2 est fonctionnel et permet l'expression du gène TK.
   2) Dès la 24ème heure, des particules virales recombinantes sont produites et permettent de transférer efficacement et de façon stable le gène TK dans les cellules de la culture.
   3) Après 5 jours, on observe une augmentation importante (mais difficilement quantifiée dans cette expérience) du nombre de clones stables obtenus.

### - EXEMPLE IV : Construction d'un plasmide unique portant à la fois la séquence pseudo-rétrovirale recombinante dépourvue du gène env. et porteuse de la thymidine kinase de HSV1, et d'un gène env. sous la dèpendance d'un promoteur en amont et d'un gène env.en aval.

Un plasmide classique est utilisé à titre de squelette de base, par exemple le PBR 322 par les méthodes décrites plus haut. Les constructions telles que représentées dans les figures 4a et 4b sont réalisées, le plasmide ainsi recombiné peut être ainsi émulsionné et enveloppé dans des liposomes ou associé à tout autre vecteur permettant de le faire pénétrer dans la cellule hôte ou dans la cellule cible.

### - EXEMPLE V : Résultats expérimentaux obtenus sur des tumeurs établies du système hôte-vecteur obtenu avec les constructions décrites dans les exemples I, II et III :

Il a été constaté que ce système hôte-vecteur ainsi construit produit des particules rétrovirales infectantes à un titre de 108 particules/ml.

Le titre a été vérifié par infection de cellules L- de souris déficientes en thymidine kinase et infectées selon la méthode décrite dans Caruso M. et al., Proc. Natl. Acad. Sci. USA, 90:7024-7028, 1993.

En conclusion, le procédé de traitement de cellules in vivo par thérapie génique à l'aide du système hôte-vecteur et les médicaments contenant à titre de principe actif ce système apparaît comme étant particulièrement avantageux au regard des techniques préexistantes sur les différents points cités plus haut, à savoir :
- la productivité des particules virales émises (au moins 1 à 2 logs au-dessus de la productivité des cellules de packaging classiques),
- le caractère opérationnel au niveau d'une mise en oeuvre industrielle de la production d'un médicament utilisant ce système puisque ce système hôte-vecteur peut utiliser des cellules hôtes cultivables en suspension et donc à grande échelle,
- une augmentation de l'efficacité du système au niveau des cellules cibles puisque la production de particules virales infectieuses porteuses du transgène est un processus itératif qui peut être répété in vivo par adjonction à l'aide d'un vecteur ou de tout moyen approprié d'une séquence d'acides nucléiques porteuse du gène env et sous la dépendance de son promoteur ; le système peut être bloqué permettant de stopper toute dissémination de particules virales recombinantes par arrêt de l'adjonction du gène env ; cela permet de contrôler, dans le cas de gènes suicides par exemple, le nombre de cellules détruites par adjonction d'analogues de nucléosides en fonction de la taille d'une tumeur éventuelle ou du nombre de cellules à détruire,
- enfin, ce système permet une grande souplesse d'utilisation tout en gardant une sécurité virale et peut être appliqué à tout autre système d'expression conditionnel de gène.

## Revendications

1. Utilisation, pour la préparation d'une composition pharmaceutique destinée au transfert d'un transgène dans une cellule eucaryote, de séquences d'acides nucléiques dont la co-expression en trans dans ladite cellule eucaryote permet à ladite cellule eucaryote de produire des particules virales infectieuses et porteuses dudit transgène.

2. Utilisation selon la revendication 1 caractérisée en ce que les séquences d'acides nucléiques sont portées par le même système de transfection.

3. Utilisation selon la revendication 1 caractérisée en ce que les séquences d'acides nucléiques sont portées par un ou plusieurs virus défectifs.

4. Utilisation selon la revendication 3 caractérisée en ce que les séquences d'acides nucléiques sont portées par un ou plusieurs adénovirus défectifs.

5. Utilisation selon la revendication 3 caractérisée en ce que les séquences d'acides nucléiques sont portées par un ou plusieurs rétrovirus défectifs.

6. Utilisation selon l'une des revendications 1 à 5 pour la préparation d'une composition pharmaceutique destinée au transfert d'un transgène dans une cellule eucaryote in vivo.

7. Utilisation selon l'une des revendications 1 à 6 caractérisée en ce que la cellule eucaryote est une cellule tumorale.

8. Utilisation selon l'une des revendications 1 à 6 caractérisée en ce que la cellule eucaryote est une cellule infectée par un virus.

9. Utilisation selon l'une des revendications 1 à 8 caractérisée en ce que le transgène est un gène dont le produit d'expression permet la transformation d'une substance non toxique en substance toxique pour la cellule eucaryote.

10. Utilisation selon la revendication 9 caractérisée en ce que le transgène code pour la thymidine kinase du virus herpès simplex de type 1.

11. Utilisation selon l'une des revendications 1 à 8 caractérisée en ce que le transgène code pour une cytokine.

12. Adénovirus défectif comprenant un acide nucléique codant pour une protéine env rétrovirale.
